**Europäisches Patentamt**

**European Patent Office**

**Office européen des brevets**

(19)

(11) Numéro de publication: **0 041 449**
**B1**

## FASCICULE DE BREVET EUROPEEN

(45) Date de publication du fascicule du brevet:
14.03.84

(21) Numéro de dépôt: 81400853.8

(22) Date de dépôt: 27.05.81

(51) Int. Cl.³: **A 61 K 37/54,** A 61 K 45/06,
A 61 K 9/08 // G02C13/00

(54) Application d'une composition ophtalmique contenant un activateur du plasminogène, au nettoyage des lentilles cornéennes.

(30) Priorité: 29.05.80 FR 8011911

(43) Date de publication de la demande:
09.12.81 Bulletin 81/49

(45) Mention de la délivrance du brevet:
14.03.84 Bulletin 84/11

(84) Etats contractants désignés:
AT BE CH DE FR GB IT LI LU NL SE

(56) Documents cités:
FR - A - 2 226 677
FR - A - 2 252 842
FR - A - 2 310 133
FR - M - 2 513
GB - A - 872 146

(73) Titulaire: **PIERRE FABRE S.A., 125, rue de la Faisanderie, F-75116 Paris (FR)**

(72) Inventeur: **Dussourd D'Hinterland, Lucien, Domaine de la Plombière, F-81100 Castres (FR)**
Inventeur: **Normier, Gérard, 23 rue Francis Poulenc, F-81100 Castres (FR)**

(74) Mandataire: **Corre, Jacques Denis Paul et al, Cabinet Regimbeau 26, Avenue Kléber, F-75116 Paris (FR)**

BUNDESDRUCKEREI BERLIN

# Application d'une composition ophtalmique contenant un activateur du plasminogéne au nettoyage des lentilles cornéennes

La présente invention concerne les applications de compositions ophtalmiques contenant à titre de principe actif un activateur du plasminogène selon le brevet français n° 7 342 981.

Ce brevet français décrit un procédé de préparation d'un activateur du plasminogène ainsi que l'activateur obtenu. Ce produit est décrit comme un médicament utilisable pour le traitement des thromboses artérielles et veineuses en particulier et est de préférence administré par injection intraveineuse ou en perfusion.

La demanderesse a maintenant découvert que cet activateur du plasminogène était utilisable dans des compositions ophtalmiques.

En particulier, la présente invention concerne l'application d'une composition caractérisée en ce qu'elle comporte à titre de principe actif un activateur du plasminogène obtenu par la mise en oeuvre du procédé selon l'une des revendications 1 à 16 du brevet français n° 7 342 981 et un véhicule acceptable en ophtalmologie à la conservation et au nettoyage de lentilles cornéennes.

Par »activateur du plasminogène obtenu par la mise en oeuvre du procédé selon l'une des revendications 1 à 16 du brevet principal français n° 7 342 981«, on entend également désigner un activateur perfectionné obtenu par la mise en oeuvre du procédé perfectionné objet du certificat d'addition n° 7 919 432.

Afin de simplifier la terminologie, dans la revendication principale du présent brevet les termes »activateur du plasminogène« désignent spécifiquement les activateurs du plasminogène qui peuvent être obtenus pa la mise en oeuvre du procédé décrit dans le brevet français n° 7 342 981 ou son addition n° 7 919 432.

Le brevet français n° 7 342 981 décrit un procédé d'obtention d'un activateur du plasminogène, caractérisé en ce que l'on traite une poudre acétonique d'organes animaux choisis parmi les poumons et reins de porcs, veaux, boeufs, chevaux, agneaux ou moutons ou les ovaires de porcins, bovins ou ovins, par:

a) mise en suspension de ladite poudre dans une solution aqueuse saline de faible force ionique au voisinage de la neutralité;

b) reprise du précipité obtenu à l'étape a dans une solution aqueuse saline de force ionique comprise entre environ 0,6 et 1 à un pH compris entre environ 3 et 5;

c) précipitation de la solution obtenue après décantation à l'étape b par addition d'un sel ou d'un solvant organique à un pH compris entre environ 3 et 5;

d) reprise du précipité obtenu à l'étape c par de l'eau ou une solution saline et précipitation par addition d'un sel à un pH voisin ou légèrement supérieur à 7;

e) reprise du précipité obtenu à l'étape d par de l'eau et dialyse de la solution jusqu'à une résistivité de l'ordre de 1000 ohm · cm à 10°C; puis

f) purification éventuelle de la solution obtenue.

Cet activateur du plasminogène est de préférence obtenu à partir d'ovaires de truies.

Quant à l'addition n° 7 919 432, elle décrit un procédé qui est un perfectionnement du précédent, dans lequel l'activateur du plasminogène est séparé des impuretés par adsorption sur de la fibrine.

Il a, en effet, été découvert que les compositions selon la présente invention étaient capables de lyser rapidement et efficacement les exsudats fibrineux apparaissant dans certaines affections de l'oeil telles que conjonctivite, uvéite et ulcérations diverses par exemple, dans certaines opérations telles que les opérations de glaucome pour empêcher l'obstruction des fistules ou même lors du fonctionnement normal de l'oeil pour l'entretien des lentilles cornéennes.

Les compositions selon la présente invention sont, de préférence, des solutions de conditionnement ou de nettoyage des lentilles cornéennes, en particulier des lentilles molles.

Les compositions selon la présente invention peuvent également comporter un autre principe actif qui augmente l'efficacité de l'activateur du plasminogène, en particulier un polysaccharide sulfaté, pour en faire un activateur du plasminogène perfectionné tel que décrit dans le certificat d'addition 7 513 932 déposé le 5 mai 1975 au nom de la demanderesse.

Les véhicules acceptables en ophtalmologie utilisables pour la réalisation des compositions selon la présente invention sont les véhicules connus. En particulier dans le cas des collyres il faut citer l'eau, en général l'eau distillée. Il est bien entendu possible de prévoir des adjuvants divers, en particulier des isotonisants tels que le chlorure de sodium ou un autre sel si nécessaire, des mélanges tampons tels que les tampons phosphates et le mélange acide borique/borate de sodium.

De même, les compositions de l'invention peuvent être utilisées, en particulier sous forme de solution, pour donner aux lentilles cornéennes souples les mêmes possibilités de nettoyage que possède physiologiquement la cornée. En effet, sur toutes les lentilles de contact souples s'accumulent au long de la journée des débris riches en fibrine qui gênent la transparence et sont une cause d'irritation. Aucun moyen actuel n'existe pour résoudre ce problème.

De la même façon, les compositions selon l'invention peuvent être utilisées pour conditionner, conserver et nettoyer les lentilles. En particulier, les compositions peuvent être utilisées pour imprégner les lentilles artificielles avant leur implantation et administrées ensuite localement pour maintenir la transparence pendant la durée de la réaction inflammatoire.

Sous forme de solutions, les compositions selon l'invention peuvent être utilisées pour conserver et rincer les lentilles cornéennes à la place des produits actuels, permettant ainsi d'éviter les altérations du polymère des lentilles (prolongation de l'usage) et de diminuer l'irritation de l'oeil (meilleure tolérance).

Les compositions selon l'invention, lysant électivement la fibrine, sont un produit de choix pour faire disparaître ces exsudats.

Les compositions selon la présente invention présentent, enfin, l'avantage d'être stables à température ordinaire.

## Revendications

1. Application d'une composition contenant à titre de principe actif un activateur du plasminogène, dans un véhicule acceptable en ophtalmologie, à la conservation et au nettoyage des lentilles cornéennes.

2. Application selon la revendication 1, caractérisée en ce que la composition se présente sous forme d'une solution.

3. Application selon l'une des revendications 1 et 2, caractérisée en ce que la composition comporte, en outre, un polysaccharide sulfaté.

4. Application selon l'une des revendications 1 à 3, caractérisée en ce que le véhicule est constitué par de l'eau distillée.

## Patentansprüche

1. Verwendung einer Zusammensetzung, die als aktiven Bestandteil einen Plasminogenaktivator in einem ophthalmologisch annehmbaren Träger enthält, zur Aufbewahrung und Reinigung von Kontaktlinsen.

2. Verwendung nach Anspruch 1, dadurch gekennzeichnet, daß die Zusammensetzung in Form einer Lösung verwendet wird.

3. Verwendung nach einem der Ansprüche 1 und 2, dadurch gekennzeichnet, daß die Zusammensetzung weiters ein sulfatiertes Polysaccarid enthält.

4. Verwendung nach einem der Ansprüche 1 bis 3, dadurch gekennzeichnet, daß der Träger destilliertes Wasser ist.

## Claims

1. Application of a composition containing by way of principle constituent a plasminogen activator, in a carrier acceptable in ophthalmology, for the care and cleaning of contact lenses.

2. Application according to Claim 1, characterised in that the composition is presented in the form of a solution.

3. Application according to either of Claims 1 and 2, characterised in that the composition has, in addition, a sulphated polysaccharide.

4. Application according to any one of Claims 1 to 3, characterised in that the carrier is constituted by distilled water.